Europäisches Patentamt

European Patent Office

Office européen des brevets

(11) Veröffentlichungsnummer: **0 362 671**

**A1**

## (12) EUROPÄISCHE PATENTANMELDUNG

(21) Anmeldenummer: 89117745.3

(22) Anmeldetag: 26.09.89

(51) Int. Cl.5: **C07H 15/04**

(30) Priorität: 05.10.88 DE 3833780

(43) Veröffentlichungstag der Anmeldung:
**11.04.90 Patentblatt 90/15**

(84) Benannte Vertragsstaaten:
**ES GR**

(71) Anmelder: **Henkel Kommanditgesellschaft auf Aktien**
**Postfach 1100 Henkelstrasse 67**
**D-4000 Düsseldorf 1(DE)**

(72) Erfinder: **Hill, Karlheinz, Dr.**
**Am Hasenbusch 1**
**D-4006 Erkrath(DE)**
Erfinder: **Biermann, Manfred, Dr.**
**Markscheiderhof 25**
**D-4330 Mülheim(DE)**
Erfinder: **Rossmaier, Henry, Dr.**
**Tannenhofweg 43**
**D-4000 Düsseldorf 12(DE)**
Erfinder: **Eskuchen, Rainer, Dr.**
**Benrather Schlossallee 36**
**D-4000 Düsseldorf(DE)**
Erfinder: **Wüst, Willi, Dr.**
**Fasanenring 32**
**D-4030 Ratingen(DE)**
Erfinder: **Wollman, Josef**
**Kloster Strasse 112**
**D-5120 Herzogenrath 3(DE)**
Erfinder: **Bruns, Andreas, Dr.**
**Weissensten 65**
**D-4018 Langenfeld(DE)**
Erfinder: **Hellmann, Dr.**
**Am Bruchhauser Kamp 15**
**D-4010 Hilden(DE)**
Erfinder: **Ott, Karl-Heinz, Dr.**
**Trillser Graben 6**
**D-4006 Erkrath(DE)**
Erfinder: **Winkle, Walter, Dr.**
**Krischerstrasse 81**
**D-4019 Monheim(DE)**
Erfinder: **Wollmann, Klaus, Dr.**
**Röntgenstrasse 29**
**D-5657 Haan(DE)**

(54) **Verfahren zur direkten Herstellung von Alkylglykosiden.**

㊼ Die aliphatischen primären Alkohole werden mit einer Glykose, insbesondere mit Glucose, in Gegenwart eines sauren Katalysators in bestimmt gehaltenen Verfahrensschritten so miteinander umgesetzt, daß in Verbesserung bekannter Verfahren der Direktsynthese und nach einer angeschlossenen zwingenden Bleichstufe besonders hellfarbige und alkalistabile Alkylglucoside resultieren. Das Verfahren kann sowohl auf Laborstufe als auch auf großtechnischer Produktionsstufe durchgeführt werden.

EP 0 362 671 A1

## Verfahren zur direkten Herstellung von Alkylglykosiden

Die Erfindung betrifft eine Weiterentwicklung des Verfahrens zur direkten Herstellung von oberflächenaktiven Alkylglykosiden, also den Acetalen aus Zuckern und aliphatischen Alkoholen, durch direkte säurekatalysierte Umsetzung der Alkohole mit den Zuckern unter Wasserabspaltung.

Der Begriff Alkylglykoside wird im folgenden für die Reaktionsprodukte aus Zuckern und aliphatischen Alkoholen verstanden, wobei als Zuckerkomponente die im folgenden als Glykosen bezeichneten Aldosen bzw. auch Ketosen im weitesten Sinne in Betracht kommen, wie z. B. die Glucose, Fructose, Mannose, Galactose, Talose, Gulose, Allose, Altrose, Idose, Arabinose, Xylose, Lyxose und Ribose. Vorzugsweise werden wegen der besseren Reaktionsfähigkeit die Aldosen verwendet. Unter den Aldosen kommt wegen ihrer leichten Zugänglichkeit und Verfügbarkeit in technischen Mengen insbesondere die Glucose in Betracht. Die nach dem Verfahren der Erfindung besonders bevorzugt hergestellten Alkylglykoside sind deshalb die Alkylglucoside. Der Begriff Alkyl in Alkylglykosid umfaßt im weitesten Sinne den Rest eines aliphatischen Alkohols beliebiger Kettenlänge, vorzugsweise eines primären aliphatischen Alkohols und insbesondere eines aus natürlichen Fetten erhältlichen Fettalkohols, so daß der Begriff gesättigte und ungesättigte Reste und deren Gemische einschließlich solcher mit verschiedenen Kettenlängen im Gemisch umfaßt. Die Begriffe Alkyloligoglykosid, Alkylpolyglykosid, Alkyloligosaccharid und Alkylpolysaccharid beziehen sich auf solche alkylierten Glykosen, in denen ein Alkylrest in Form des Acetals an mehr als einen Glykoserest, also an einen Poly- oder Oligosaccharidrest gebunden ist. Diese Begriffe werden als untereinander gleichbedeutend angesehen. Dementsprechend ist ein Alkylmonoglykosid das Acetal eines Monosaccharids. Da man bei der säurekatalysierten Umsetzung von Zuckern und Fettalkoholen im allgemeinen Gemische erhält, werden im folgenden unter dem Begriff Alkylglykosid sowohl Alkylmonoglykoside als auch Alkylpoly(oligo)glykoside und insbesondere Mischungen daraus -einschließlich eventueller Nebenkomponenten - verstanden, sofern es nicht ausdrücklich auf die strukturellen Unterschiede ankommt.

Die oberflächenaktiven Alkylglykoside sind als Waschmittelrohstoffe bereits seit über 50 Jahren bekannt. So beschreibt die österreichische Patentschrift Nr. 135 333 die Herstellung von Laurylglucosid und Cetylglucosid aus Acetobromglucose und dem jeweiligen Fettalkohol in Gegenwart einer Base. Auch die Direktsynthese aus Glucose und Laurylalkohol mit Chlorwasserstoff als saurem Katalysator ist dort beschrieben. Nach der Lehre der deutschen Patentschrift 611 055 werden Alkylglucoside aus Pentaacetylglucose und dem Fettalkohol in Gegenwart von wasserfreiem Zinkchlorid hergestellt. Die Maltoside und Lactoside der aliphatischen Alkohole mit mehr als 8 Kohlenstoffatomen und ihre Verwendung als Tenside sind aus der deutschen Patentschrift Nr. 593 422 bekannt. Beispielsweise ist in dieser Veröffentlichung angegeben, daß Cetylmaltosid die Waschwirkung von Seife, die damals das wichtigste Tensid war, verbessert, was mit der Wirkung des Cetylmaltosids als Kalkseifendispergator erklärt wird. Aus den 60er und 70er Jahren stammen mehrere Vorschläge für die verbesserte Herstellung von Alkylglykosiden entweder durch direkte Umsetzung der Glykose, meist Glucose, mit einem Überschuß des Alkohols und einer Säure als Katalysator, oder unter Mitverwendung eines niederen Alkohols oder Glykols als Lösungsmittel und Reaktionspartner zu einem primären Reaktionsprodukt, aus dem durch Umacetalisierung mit dem längerkettigen Alkohol erst das oberflächenaktive Alkylglykosid gewonnen wird. In der US-Patentschrift 3,450,690 (Gibbons et al) wird ein Verfahren der Direktsynthese von Alkylglucosiden, allerdings mit $C_1$- bis $C_8$-Alkanolen, beschrieben, wobei Synthese-Nebenprodukte bzw. - Verunreinigungen, die im alkalischen Medium unerwünschte Verfärbungen hervorrufen, dadurch aus dem Rohprodukt entfernt werden, daß man dieses in wäßriger Lösung unter Erwärmen mit anorganischen oder organischen Basen wie z. B. Natriumhydroxid, Natriummethylat, Calciumhydroxid, Bariumhydroxid, Bariummethylat oder stark basischen Aminen behandelt. Dabei soll der saure Katalysator (z. B. Schwefelsäure) nicht nur neutralisiert werden, sondern es wird ein alkalischer pH-Wert von wenigstens 8 eingestellt. Nach dem Erhitzen auf Temperaturen zwischen 50 und 200 °C fallen die Verunreinigungen aus. Daraufhin wird abfiltriert und der Alkoholüberschuß abdestilliert. Als wäßrige Lösung wird in dieser Literaturstelle die Mischung aus dem Überschuß des alkoholischen Reaktionspartners und des bei der Reaktion entstandenen Wassers verstanden. In einigen Beispielen wird der überschüssige Alkohol (Ethanol) entfernt und zum Teil durch Wasser ersetzt. Nach dem Abfiltrieren des unlöslichen Niederschlages wird das Filtrat durch Behandeln mit Aktivkohle aufgehellt. Als äquivalente Maßnahme zur Aktivkohlebehandlung wird auch das Bleichen mit Wasserstoffperoxid erwähnt. Vorzugsweise wird als Base Calciumhydroxid verwendet. In der US-Patentschrift 3,839,318 (Mansfield et al) wird ein Verfahren der direkten Glucosidierung von langkettigen Alkoholen beschrieben, wobei die Reaktionsgeschwindigkeit durch Steuerung der Reaktionstemperatur und der Katalysatorkonzentration so geführt wird, daß das sich bildende Reaktionswasser aus dem Reaktionsgemisch möglichst schnell via azeotrope Destillation entfernt wird. Dabei wird vorzugsweise ein Kohlenwasserstoff als Lösungsmittel, z. B. Hexan oder Heptan, zugesetzt, um die rasche azeotrope Destillation des Wassers zu ermöglichen. Anschließend wird mit einer wäßrigen Lösung von Natriumhydroxid neutralisiert, wobei auch alkalische pH-Werte eingestellt werden können. Danach wird der überschüssige Alkohol auf die übliche Weise durch Destillation entfernt. Das Überführen des Reaktionsprodukts in eine wäßrige Paste und Bleichen dieser Paste mit Natriumperborat wird ebenfalls beschrieben.

Nach den Angaben der europäischen Patentanmeldung 132 046 (Procter & Gamble, Letton) wird bei einem Verfahren der Direktsynthese der saure Katalysator mit einer organischen Base neutralisiert, wobei ein enger pH-Wert-Bereich in der Nähe des Neutralpunkts (pH 6,6 bis 7, vorzugsweise 6,7 bis 6,8) eingestellt wird. Als organische Basen werden entweder die Alkali(Na, K, Li)- oder Erdalkali-(Ba, Ca)- oder Aluminiumsalze von schwachen niedermolekularen Säuren, z. B. Natriumacetat, oder die entsprechenden Alkoholate, z. B. Natriumethylat, eingesetzt.

In der europäischen Patentanmeldung 96 917 (Procter & Gamble, Farris) wird ein verbessertes Verfahren der säurekatalysierten Direktsynthese beschrieben, wobei man ein Monosaccharid, vorzugsweise Glucose, kontinuierlich oder portionsweise so zu der Mischung aus Fettalkohol und Katalysator bei 80 bis 150 °C hinzufügt, daß nie mehr als 10 nicht umgesetztes Monosaccharid im Reaktionsgemisch vorhanden sind.

Die europäische Patentanmeldung 77 167 (Rohm & Haas, Arnaudis) schlägt zur Verbesserung der Farbqualität von oberflächenaktiven Alkylglykosiden vor, daß man bei ihrer Herstellung einen üblichen sauren Katalysator zusammen mit einem sauren Reduktionsmittel aus der Gruppe der phosphorigen Säure, hypophosphorigen Säure, schwefeligen Säure, hyposchwefeligen Säure, salpetrigen Säure und/oder hyposalpetrigen Säure bzw. der entsprechenden Salze, verwendet.

Hellfarbige $C_3$- bis $C_5$-Alkylglucoside werden nach der Lehre der europäischen Patentanmeldung 102 558 (BASF, Lorenz et al) dadurch erhalten, daß man sie in Gegenwart eines sauren Katalysators und mindestens dazu äquivalenten Mengen eines Alkalimetallsalzes einer Borsäure, vorzugsweise Natriumperborat, herstellt.

Schließlich wird in der europäischen Patentanmeldung 165 721 (Staley, McDaniel et al) vorgeschlagen, die wäßrige Lösung eines oberflächenaktiven Alkylpolyglucosids zunächst mit einem Oxidationsmittel, vorzugsweise mit einer Wasserstoffperoxidlösung, und anschließend mit einer Schwefeldioxidquelle, z. B. einer wäßrigen Lösung von Natriumbisulfit, zu behandeln. Die so erhaltenen Produkte sollen auch nach längerem Lagern farbstabil sein.

Bei der Herstellung von Tensidrohstoffen war man schon immer bestrebt, möglichst farblose Produkte zu erhalten. Farbige Verunreinigungen oder zunächst farblose Produkte, die sich beim Lagern verfärben, werden häufig als minderwertig oder unbrauchbar eingestuft, wenn mit ihnen keine ästhetisch befriedigenden Mischungen erzielt werden können. Bei der Weiterverarbeitung von Tensidrohstoffen spielt die Farbstabilität im alkalischen Medium eine besondere Rolle. Es ist zwar häufig möglich, technische Tensidrohstoffe durch Bleichen, beispielsweise mit wäßrigen Wasserstoffperoxidlösungen, in hellfarbige Produkte, die auch beim Lagern und im alkalischen Medium hellfarbig bleiben, überzuführen. Bei den oberflächenaktiven Alkylglykosiden, die bisher bekanntgeworden sind, versagt allerdings diese Bleichbehandlungi denn auch scheinbar aufgehellte Produkte nehmen wieder eine dunkelbraune Färbung an, wenn sie nach dem Bleichen mit wäßrigem Alkali bei erhöhter Temperatur behandelt werden. Den bekannten Herstellungsverfahren für Alkylglykoside, die auch eine verbesserte Farbqualität und Lagerstabilität anstreben, haftet der Nachteil an, daß man dabei entweder zusätzliche chemische Wirkstoffe während des Herstellungsverfahrens zusetzen muß, oder aber, daß ein solcher Zusatz für eine Nachbehandlung des Reaktionsproduktes erforderlich ist. Die vorliegende Erfindung setzt sich zum Ziel, ein neues Verfahren zur Herstellung von oberflächenaktiven Alkylglykosiden nach der sogenannten Direktsynthese bereitzustellen, wobei durch geeignete Wahl und Ausgestaltung der Verfahrensparameter dafür gesorgt wird, daß das im letzten Verfahrensschritt gebleichte Produkt beim Lagern und Weiterverarbeiten die erreichte Hellfarbigkeit auch unter alkalischen Bedingungen bei erhöhter Temperatur beibehält. Weiter ist es ein Ziel der Erfindung, die Verfahrensschritte so zu gestalten, daß man mit einem Minimum an chemischen Reaktionspartnern und einem Minimum an Verfahrensmaßnahmen auskommt. Schließlich ist es ein Ziel der Erfindung, die Verfahrensschritte so auszuwählen, daß eine Übertragung des Verfahrens in den großtechnischen Maßstab ohne scaling-up-Probleme möglich ist und sich das neue Verfahren für die Herstellung von oberflächenaktiven Alkylglykosiden in solchen Mengen eignet, daß das Verfahrensprodukt als Tensidrohstoff von der Waschmittelindustrie verarbeitet werden kann.

Es wurde nun gefunden, daß sich diese und weitere Ziele durch eine neuartige Kombination von sowohl an sich bekannten als auch neuen Verfahrensmerkmalen zu einem neuen Verfahren der Direktsynthese erreichen lassen.

Das erfindungsgemäße Verfahren betrifft die direkte Herstellung von Alkylglykosiden durch Acetalisierungsreaktion von höheren aliphatischen primären Alkoholen mit Glykosen, insbesondere mit Glucose, in Gegenwart eines sauren Katalysators, rascher Entfernung des Reaktionswassers, Neutralisation des Katalysators mit einer Base, Abdestillation des Alkoholüberschusses und Überführen des Reaktionsproduktes in eine wäßrige Paste und Blechen dieser Paste, wobei der aliphatische Alkohol im molaren Überschuß zur Glykose eingesetzt wird, und wobei die Bildung und Abführung des Reaktionswassers unter Vakuum erfolgt und Reaktionstemperaturen über 80 °C angewendet werden. Das Verfahren ist dadurch gekennzeichnet, daß man:

a) Mischungen aus aliphatischem primärem Alkohol, Glykose und saurem Katalysator bei erhöhter Temperatur, erzeugt und zur Reaktion bringt, wobei man entweder

(i) eine Teilmenge des Alkohols mit dem Katalysator vorlegt, die Mischung erwärmt, und dann eine erwärmte Suspension der Glykose in der restlichen Alkoholmenge portionsweise oder kontinuierlich zu der Alkohol/Katalysatormischung zudosiert und dabei im Vakuum das entstehende Reaktionswasser abdestilliert, oder

(ii) eine Mischung des gesamten Alkohols und der Glykose vorlegt, erwärmt und den sauren Katalysator zur erwärmten Mischung hinzugibt, dann ein Vakuum anlegt und weiter bis zum Einsetzen der Reaktion erwärmt und das entstehende Reaktionswasser abdestilliert,

b) dabei die Ansatzverhältnisse so wählt, daß das Molverhältnis Glykose zu aliphatischem Alkohol bei 1 : 2 bis 1 : 10, vorzugsweise 1 : 3 bis 1 : 6 liegt,

c) das Reaktionsgemisch bei dieser Temperatur und diesem Unterdruck so lange hält, vorzugsweise unter Durchmischung, bis das Reaktionswasser vollständig entfernt ist,

d) anschließend das Reaktionsgemisch auf ca. 90 ° abkühlt, dann eine organische oder anorganische basische Alkali-, Erdalkali- oder Aluminium- bzw. Alkali/Aluminiumverbindung in solchen Mengen hinzufügt, daß sich über die Neutralisation des sauren Katalysators hinaus ein pH-Wert von wenigstens 8, vorzugsweise 8 bis 10 einstellt, und vorzugsweise erst danach Normaldruck herstellt,

e) daß man vorzugsweise ohne vorherige Filtration den überschüssigen Alkohol aus dem alkalischen Gemisch im Vakuum auf an sich übliche, das Reaktionsprodukt schonende Weise auf einen Wert von unterhalb 5 Gew.-% des Reaktionsproduktes abdestilliert, und

f) daß man anschließend auf ca. 105 °C bis 130 °C abkühlt und durch Hinzugabe von Wasser eine 30- bis 70prozentige Paste erzeugt, die man durch vorzugsweise portionsweises Hinzufügen von Aktivsauerstoffverbindungen, vorzugsweise Wasserstoffperoxid, ca. 0,1 bis 5 Stunden bei ca. 80 °C rührt, wobei man gegebenenfalls durch Hinzufügen von Alkali, vorzugsweise Natronlauge, dafür sorgt, daß der pH-Wert während dieses Bleichprozesses bei 8 bis 10 bleibt.

Das Reaktionsprodukt wird in Form einer farblosen bis gelblichen wäßrigen Paste erhalten. Es wurde überraschenderweise gefunden, daß diese Paste beim Lagern und insbesondere auch beim Weiterverarbeiten im alkalischen Milieu die ursprüngliche Farbqualität nahezu unverändert beibehält. Die Farbstabilität des Produkts wird durch einen einfachen Test festgestellt. Dazu wird eine Probemenge des Produkts mit Wasser zu einer ca. 50%igen Paste vermischt und bei Normaltemperatur mit konzentrierter Natronlauge versetzt, so daß sich ein pH-Wert von ca. 12 bis 13 einstellt. Dann wird 30 Minuten lang auf 100 °C erhitzt. Bei den Ansätzen mit Verfahrensprodukten trat nach dieser Behandlung keine oder keine wesentliche Farbänderung ein. Die Farbzahlen für die Produkte wurden nach der Methode von KLETT bestimmt (5%ige Lösung in Wasser/Isopropylalkohol 1 : 1, 1 cm-Küvette, Blaufilter). Mit dieser Testmethode können Langzeitlagerversuche des pastenförmigen Produkts unter üblichen Bedingungen sowie Weiterverarbeitungsverfahren des gelagerten Produkts zu insbesondere Wasch- und Reinigungsmitteln und den dabei auftretenden alkalischen Bedingungen zuverlässig simuliert werden. Die Verfahrensprodukte besitzen vorzugsweise Klettzahlen von weniger als 35.

Als Glykose wird bei dem erfindungsgemäßen Verfahren vorzugsweise die Glucose verwendet. Handelsübliche Glucose enthält häufig 1 Mol Kristallwasser. Diese kristallwasserhaltige Glucose kann ohne weiteres verwendet werden. Dann sollte allerdings zweckmäßigerweise dieses Kristallwasser zusätzlich, und zwar vor dem Inkontaktbringen mit dem Katalysator aus dem Reaktionsmilieu durch thermische Maßnahmen entfernt werden. Nachdem aber auch wasserfreie Glucose in großen Mengen am Markt erhältlich ist, wird bevorzugt wasserfreie Glucose als feinteiliges Pulver eingesetzt.

Als Katalysatoren sind generell alle sauren Verbindungen einschließlich der sogenannten Lewis-Säuren, welche die Acetalisierungsreaktion zwischen Fettalkohol und Zuckermolekül katalysieren, geeignet. Davon gelten die Schwefelsäure, Phosphorsäure, aliphatische und/oder aromatische Sulfonsäuren, vorzugsweise Paratoluolsulfonsäure und die sulfosauren Ionenaustauscherharze als besonders geeignet. Für das vorliegende Verfahren werden die Schwefelsäure und insbesondere die Paratoluolsulfonsäure, die eine geringere korrodierende Wirkung gegenüber Geräten und Leitungen aus Stahl hat, als Katalysator bevorzugt. Saure Ionenaustauscherharze sind im vorliegenden Fall ebenfalls geeignet, wenn die Abtrennung des Katalysators nach der Acetalisierung der Glykose vorgesehen ist. In einem solchen Falle wird vorzugsweise eine geeignete basische Verbindung nach der Abtrennung des sauren Austauscherharzes hinzugegeben, um das Gemisch auf pH 8 bis 10 einzustellen.

Die Ansatzbedingungen für die drei Komponenten aliphatischer Alkohol, Glykose und Katalysator sind in weiten Grenzen variierbar. So ist es nach einer Variante des erfindungsgemäßen Verfahrens möglich, eine Mischung der Gesamtmengen aller Komponenten vorzulegen und durch Erwärmen die Reaktion einzuleiten. Nach einer anderen Variante wird eine Teilmenge des Alkohols mit dem Katalysator vorgelegt und die erwärmte Suspension der Glykose in der restlichen Alkoholmenge nach und nach hinzugefügt. Dabei gibt man im Falle von Laboransätzen einer portionsweisen und im Falle von großtechnischen Ansätzen einer kontinuierlichen Zugabe den Vorzug. Vorzugsweise werden bei der Dosierung die Zeitintervalle zwischen den Dosierportionen so gewählt, daß ständig eine im wesentlichen klare Phase vorliegt, d. h., daß die Menge der nicht umgesetzten Glykose im Reaktionsgemisch gering, d. h. bei nicht mehr als 10 %, gehalten wird. Auch das Ansatzverhältnis Glykose zu aliphatischem Alkohol kann in weiten Grenzen variiert werden. Auf diese Weise ist es möglich, den Verteilungsgrad zwischen Alkylmonoglykosid und Alkyloligoglykosiden im Reaktionsprodukt zu steuern.

Bei Laboransätzen und insbesondere bei den Ansätzen im großtechnischen Maßstab wurde gefunden, daß die Feindispergierung der Glykose im insbesondere langkettigen Alkohol einen wesentlichen positiven Einfluß auf die Qualität des Reaktionsproduktes hat. Diese Feindispergierung wird dadurch erreicht, daß man die feinpulverige Glykose, vor allem die Glucose, gegebenenfalls nach einer Feinmahlung, mit dem Alkohol intensiv vermischt. Für Laboransätze haben sich dafür die Verwendung eines hochtourigen üblichen Laborrührers oder aber eine Ultraschallbehandlung als geeignet erwiesen. Bei großtechnischen Ansätzen werden zur Feindispergierung mit Vorteil Inline-Mischer, beispielsweise ein Stator/Rotor-Mischer verwendet. Diese Feindispergierungsmaßnahme hat den erwünschten Nebeneffekt, daß sich dabei die Suspension erwärmt.

Während der Bildung und Abführung des Reaktionswassers wird ein Vakuum von etwa 10 bis 50 mbar angelegt. Während der Reaktion wird die Mischung erwärmt und vorzugsweise ständig durchmischt, was bei Laboransätzen durch einfaches Rühren geschieht, während bei großtechnischen Ansätzen dieses Durchmischen und Erwärmen durch Umpumpen über einen externen Flüssigkeitskreislauf mit einem Wärmeaustauscher erfolgt. Beim Zuführen der Wärmeenergie, die zur Aufrechterhaltung der Reaktionstemperatur benötigt wird, ist es wesentlich, daß zwischen Reaktorwand und Reaktionsgemisch nur eine geringe Temperaturdifferenz vorhanden ist, damit Überhitzungen vermieden werden. Um diese geringe Temperaturdifferenz einzustellen, genügt es bei Ansätzen im Labor, ein übliches Ölbad mit Thermostat zu verwenden und gleichzeitig das Reaktionsgemisch kräftig zu rühren. Bei Ansätzen im technischem Maßstab hat sich als besonders vorteilhaft erwiesen, die Wärmeenergie über einen externen Kreislauf, vorzugsweise bestehend aus einer Pumpe und einem Wärmeaustauscher, vorzunehmen. Zu diesem Zweck wird ständig ein Teil des Reaktionsgemisches über eine Rohrleitung abgezogen, im Wärmeaustauscher erwärmt und wieder in den Reaktor zurückgeführt. Auf diese Weise ist es möglich, hohe Reaktorwandtemperaturen, d. h. solche von mehr als 125 $^\circ$C zu vermeiden und damit eine negative Auswirkung der Temperaturführung auf die Farbwerte des Endproduktes zu verhindern.

Die aliphatischen primären Alkohole, die erfindungsgemäß zur Umsetzung gebracht werden, können an sich beliebige Kettenlängen, d. h. solche von 1 bis etwa 30 Kohlenstoffatomen, aufweisen. Um oberflächenaktive Reaktionsprodukte, die als Tensidrohstoffe in Wasch- und Reinigungsmitteln eingesetzt werden können, zu erhalten, werden aliphatische primäre Alkohole mit 8 bis 20 Kohlenstoffatomen, insbesondere solche mit 12 bis 18 Kohlenstoffatomen, bevorzugt. Diese höheren aliphatischen Alkohole werden vorzugsweise aus technischen Fetten hergestellt. Selbstverständlich ist es aber auch möglich, synthetische primäre Alkohole wie z. B. die sogenannten Oxoalkohole nach dem erfindungsgemäßen Verfahren einzusetzen.

Arbeitet man nach der Dosierungsvariante des Verfahrens, dann legt man vorzugweise 30 bis 70 Gew.-% des Alkohols zusammen mit dem Katalysator vor, erwärmt die Mischung auf 100 bis 120 $^\circ$C und gibt dann die Glykose als Suspension der erwärmten restlichen Alkoholmenge hinzu, wobei man diese Zugabe vorzugsweise kontinuierlich unter Vakuum durchführt. Das entstehende Reaktionswasser wird ständig abdestilliert. Das Ende der Reaktion gilt dann als erreicht, wenn sich kein weiteres Reaktionswasser abscheidet. Um die Menge des Reaktionswassers zu bestimmen und so das Reaktionsende festzustellen, kann das Wasser beispielsweise durch Ausfrieren in einer Kühlfalle aufgefangen werden. Bei vorgegebenen Ansatzmengen und Reaktionsbedingungen kann somit die Reaktionszeit zuverlässig bestimmt werden, ohne daß jedes Mal das Auffangen und Messen des Reaktionswassers erforderlich wäre.

Bei der ebenfalls bevorzugten Verfahrensvariante, bei der man die Gesamtmenge des Ansatzes vorlegt, wird vorzugsweise so verfahren, daß man zunächst die Mischung aus Alkohol und Glykose vorlegt, diese Mischung unter Rühren erwärmt, d. h. bis ca. 80 °C Sumpftemperatur, und dann den sauren Katalysator zu der erwärmten Mischung hinzugibt. Danach wird ein Vakuum angelegt und weiter bis auf ca. 100 bis 120 °C erwärmt und das entstehende Reaktionswasser abdestilliert.

Da man, wie bereits ausgeführt, beim erfindungsgemäßen Verfahren die Alkohole mit einem weiten Kettenlängenbereich verwenden kann, läßt sich das Ausmaß des Vakuums auch so einstellen, daß dabei der Siedepunkt des Alkohols um wenigstens 30 °C gesenkt wird. Für die Umsetzung der langkettigen Fettalkohole mit $C_{12}$- bis $C_{18}$ Kohlenstoffatomen wird das Vakuum vorzugsweise auf einen Wert von 10 bis 50 mbar eingestellt.

Bei den als Alkoholkomponente besonders wichtigen höheren aliphatischen primären $C_{12}$- bis $C_{18}$-Alkoholen handelt es sich vorzugsweise um gesättigte und insbesondere um geradkettige Alkohole, wie sie durch die Hydrierung von nativen Fettsäuren im technischen Maßstab erhalten werden können. Typische Vertreter der höheren aliphatischen Alkohole, die nach dem erfindungsgemäßen Verfahren verwendet werden können, sind z. B. die Verbindungen n-Dodecylalkohol, n-Tetradecylalkohol, n-Hexadecylalkohol, n-Octadecylalkohol, n-Octylalkohol, n-Decylalkohol, Undecylalkohol, Tridecylalkohol. Da die Fettalkohole bevorzugt aus natürlichen Fettquellen stammen, kommen üblicherweise auch Gemische technischer Fettalkohole als Reaktionspartner in Betracht. Neben den eigentlichen Fettalkoholen sind auch verzweigtkettige primäre Alkohole, wie z. B. die sogenannten Oxoalkohole für die Umsetzung geeignet. Typische Oxoalkohole sind z. B. die Verbindungen $C_{12}/C_{13}$-Alkanol mit ca. 25 % hauptsächlich 2-Methylverzweigung (Dobanol 23), und der entsprechende $C_9$-$C_{11}$-Alkanol (Dobanol 91). Allerdings liegt ein Schwerpunkt des Verfahrens bei der Herstellung von Tensiden, die ausschließlich aus nachwachsenden Rohstoffen herstellbar sind.

Als basische Alkali-, Erdalkali- oder Aluminium- bzw. Alkali/Aluminiumverbindungen, die organisch oder anorganisch sein können, eignen sich beispielsweise Calciumhydroxid, Calciumoxid, Magnesiumhydroxid, Magnesiumoxid, die Zeolithe NaA oder NaX, vorzugsweise in Kombination mit Calciumhydroxid, wasserfreies Natriumcarbonat, Kaliumcarbonat, Magnesium- und Calciumcarbonat, Natriummethylat, Natriumethylat, Magnesiummethylat, Magnesiumethylat, Natrium- bzw. Magnesiumpropylat oder -butylat, d. h. die Alkoholate von niedrigsiedenden Alkoholen, vorzugsweise $C_1$-$C_4$-Alkoholen. Als besonders bevorzugte anorganische basische Verbindung kommt Magnesiumoxid in Betracht, während als besonders bevorzugte organische Base ein Magnesiumalkoholat, insbesondere das Ethanolat des Magnesiums eingesetzt wird. Dabei können sowohl das Magnesiumoxid als auch das Magnesiumalkoholat partiell, d. h. bis zur Hälfte des Mol-Gewichts durch gepulvertes Natriumhydroxid in äquivalenten Mengen ersetzt werden.

Es gilt als ein besonderes Merkmal des Verfahrens, daß man die Zusätze der basischen Verbindungen so steuert, daß über eine Neutralisation des sauren Katalysators hinaus ein Überschuß der basischen Verbindung vorliegt, so daß das Reaktionsgemisch deutlich basisch reagiert und deshalb vorzugsweise einen pH-Wert zwischen 8 und 10 aufweist. Der pH-Wert wird in einer 10%igen wäßrig/alkoholischen Emulsion einer Probemenge mit einem üblichen pH-Meßgerät gemessen.

Die Abdestillation des Alkoholüberschusses erfolgt auf eine das Reaktionsprodukt schonende Weise durch die geeignete Wahl von Vakuumdestillations-Methoden. Dabei wird bei einem Vakuum im Bereich von 0,01 bis 1 mbar destilliert. An sich gehört zur produktschonenden Destillation auch das Einstellen einer möglichst niederen Sumpftemperatur, worunter man die Temperatur des siedenden Gemisches versteht. Es hat sich jedoch im vorliegenden Fall überraschenderweise als vorteilhaft gezeigt, de Reaktionsmischung bis auf eine Sumpftemperatur von 160 bis 180 °C, insbesondere von 160 bis 170 °C, zu erhitzen, und zwar unabhängig davon, ob ein derartig hoher Wert bei der vorgegebenen Vakuumleistung zur Abdestillation des überschüssigen Alkohols notwendig wäre. Zwar führt eine derart hohe Sumpftemperatur zunächst unmittelbar zu einem Rohprodukt mit verschlechterter Farbqualität. Es konnte jedoch in unerwarteter Weise gezeigt werden, daß gerade die bei der hohen Sumpftemperatur behandelten Produkte nach dem Bleichen eine hellere Farbe und eine bessere Alkalistabilität im Sinne des oben angegebenen Tests besitzen als solche Produkte, die bei niederen Sumpftemperaturen behandelt worden waren und vor dem Bleichen eine scheinbar bessere Farbqualität aufwiesen. Es ist infolgedessen ein weiteres wichtiges Merkmal des Verfahrens, die Reaktionsmischung während der Verfahrensstufe der Entfernung des Alkoholüberschusses im Hochvakuum auf die hohe Sumpftemperatur von ca. 160 bis 180 °C zu bringen, selbst wenn diese hohe Sumpftemperatur nicht für die Abdestillation des Alkoholüberschusses erforderlich wäre, was bei den kürzerkettigen Fettalkoholen der Fall ist.

Für die Destillation von Laboransätzen werden zur Entfernung des Alkoholüberschusses übliche Vakuumdestillationsgeräte benutzt. Bei technischen Ansätzen im Produktionsmaßstab wird die Abdestillation des Alkohols vorzugsweise nach einem zweistufigen Verfahren durchgeführt, wenn es sich bei dem Fettalkohol um einen solchen aus dem Kohlenstoffkettenlängenbereich 12 bis 20 handelt, wobei man in einer ersten Stufe eine Abreicherung des Fettalkoholanteils auf Werte von ca. 40 bis ca. 20 % mit einem Dünnschichtverdampfer oder einem Fallfilmverdampfer durchführt, und wobei diese erste Stufe auch zur Entgasung des Reaktionsgemisches dient. In einer zweiten Stufe wird mit einem Kurzwegverdampfer bzw. einem Dünnschichtverdampfer die weitere Fettalkoholabreicherung auf den gewünschten Endwert eingestellt. Dieser Endwert kann, bezogen auf das Endprodukt, bei Werten unterhalb 0,5 Gew.-% liegen, wenn das Produkt praktisch frei von dem Fettalkohol sein soll. Für den Fall, daß gezielt Fettalkoholanteile im Endprodukt erwünscht sind, können diese Fettalkoholanteile bei 3 bis 5 Gew.-% eingestellt werden. Es hat sich gezeigt, daß Verfahrensendprodukte mit einem Fettalkoholanteil von über 2 Gew.-%, vorzugsweise 3 bis 5 Gew.-%, gewisse anwendungstechnische Vorteile besitzen.

Für die schonende Auftrennung von temperaturempfindlichen Substanzgemischen gilt generell, daß sich zur schonenden Verdampfung bei reduziertem Druck Fallfilmverdampfer und insbesondere Dünnschichtverdampfer besonders gut eignen, weil sich in diesen Geräten extrem kurze Verweilzeiten bei den erforderlichen höheren Temperaturen erreichen lassen. In dem vorliegenden Fall eignen sich zur Abreicherung des überschüssigen Fettalkohols mit 10 bis 18 Kohlenstoffatomen vom Alkylglykosid mit besonders guten Tensideigenschaften vor allem Dünnschichtverdampfer. Als Dünnschichtverdampfer bezeichnet man solche Verdampfer, in denen ein hochviskoses schwer siedendes Gemisch auf eine beheizte Wand aufgegeben und dort durch rotierende Wischelemente mechanisch verteilt wird. Dabei werden dünne Flüssigkeitsschichten bzw. Flüssigkeitsfilme erzeugt, und die Filmoberflächen werden ständig erneuert. Die entstehenden Dämpfe strömen entgegen dem Fluß des Produktfilms und verlassen den Verdampfer in den außen angeordneten Kondensator. Im Dünnschichtverdampfer wird im allgemeinen bei Drukken von nur einigen mbar gearbeitet und die Verweildauer für das Produkt beträgt nur wenige Sekunden. In einer zweistufigen Anlage, wie sie in dem erfindungsgemäßen Verfahren bevorzugt benutzt wird, fungiert das erste Verdampfergerät auch als Vorentgaserstufe für das in zweiter Stufe benutzte Verdampfergerät. Gase, die in der viskosen Flüssigkeit gelöst sind, werden so im Zuge der Abreicherung des Reaktionsproduktes an überschüssigem Fettalkohol im ersten Dünnschichtverdampfer aus der Flüssigkeit entfernt. Bei dem als zweites Verdampfergerät auch bevorzugt eingesetzten Kurzwegverdampfer handelt es sich im Prinzip um einen Wischfilmverdampfer mit einem im Verdampfer eingebauten Kondensator. Diese Geräte eignen sich zur Destillation hochsiedender und temperaturempfindlicher Produkte im Bereich $10^{-1}$ bis $10^{-4}$ mbar. Ähnlich wie bei dem Dünnschichtverdampfer wird auch bei dem Kurzwegverdampfer die Flüssigkeit mechanisch auf der Heizfläche durch Wischer verteilt. Erfindungsgemäß wird im Kurzwegverdampfer bzw. Dünnschichtverdampfer als zweiter Stufe der überschüssige Alkohol auf praktisch beliebige Restgehalte, die unter 1 % liegen können, entfernt. Die Zweistufenanordnung der Verdampfergeräte gestattet hohe Durchsätze in Verbindung mit der gezielten Einstellung des erwünschten Restgehaltes an Fettalkohol im Endprodukt. Für technische Zwecke lassen sich Dünnschicht- und Kurzwegverdampfer so dimensionieren, daß Durchsätze von bis zu 300 kg/qm und Stunde ohne weiteres möglich sind. Die erfindungsgemäß bevorzugte Verfahrensvariante mit der zweistufigen Fettalkoholabreicherungsanlage läßt sich prinzipiell auch in der passenden Dimensionierung für die Aufarbeitung von Laboransätzen verwenden.

Die erfindungsgemäß hergestellten Alkylglykoside stellen Gemische dar, die im wesentlichen aus Alkylmonoglykosid und den Alkyloligoglykosiden, hier im wesentlichen beschränkt auf Di- und Triglykoside, und geringen Anteilen an Tetra- und Pentaglykosiden, bestehen. Die Verteilung zwischen Mono- und Oligoglykosiden in dem Verfahrensprodukt ergibt einen rechnerischen Oligomerisierungsgrad, der zwischen 1 und 5 liegt. Vorzugsweise wird das Verfahren so geführt, daß der Oligomerisierungsgrad zwischen 1 und 1,5 liegt, wobei die Menge an Alkylmonoglykosid, bezogen auf die Gesamtmenge, aus Alkylmonoglykosid und Alkyloligoglykosid deutlich über 70 Gew.-% liegt. (Zur Definition des Oligomerisierungsgrades siehe Paul J. Flory, Principles of Polymer Chemistry, Cornell University Press, Ithaca, New York, 1953, Seiten 35 bis 37). Die Gesamtmenge der übrigen Nebenbestandteile liegt meist unter 20 Gew.-%. Von diesen Nebenbestandteilen ist der Fettalkoholanteil variabel, da er von der Intensität der Fettalkoholabdestillation abhängt. Die Restalkoholmenge ist in den Verfahrensprodukten auf einen bevorzugten Bereich von 0, 2 bis 5 Gew.-%, insbesondere 0 ,5 bis 2,5 Gew.-%, eingestellt. Die Restmengen an nicht umgesetzter Glykose liegen unterhalb von 1 %. Die Anteile an polymerer Glucose im Verfahrensprodukt liegen bei 2 bis 20, vorzugsweise 5 bis 20 Gew.-%. Die Mengen der Neutralisationsprodukte aus saurem Katalysator und basischer Verbindung und eventuellem Überschuß an dieser basischen Verbindung im Verfahrensprodukt liegen bei 0,5 bis 1,5 Gew.-%.

Diese Mengenangaben beziehen sich auf das Reaktionsprodukt, wie es unmittelbar nach der Abdestillation des Fettalkoholüberschusses vorliegt. Das eigentliche Verfahrensprodukt ist die daraus durch Behandeln mit warmem Wasser und Bleichen mit Aktivsauerstoffverbindungen, insbesondere Wasserstoffperoxid resultierende wäßrige Paste mit 30 bis 60 Gew.-% Wasseranteil. Die Menge der Aktivsauerstoff-Verbindung beträgt dabei im allgemeinen 0,2 bis 1,5 Gew.-%, berechnet als $H_2O_2$ und bezogen auf die Menge des Produkts nach der Alkoholabtrennung. Da bei dem Bleichvorgang der pH-Wert abnimmt, wird zusammen mit der Perverbindung eine Base, z. B. Natriumhydroxid, zugesetzt, um pH-Werte zwischen 8 und 10 aufrechtzuerhalten. Die resultierende Lösung bzw. Paste enthält vorzugsweise die aus der Neutralisation des Katalysators und dem Bleichvorgang stammenden, nicht abgetrennten Salze. Es hat sich gezeigt, daß auf vielen Anwendungsgebieten diese nicht abgetrennten Salze in der wäßrigen Alkylglykosid-Paste nach Menge und Art nicht stören. Vielmehr handelt es sich hier um Verbindungen, die ohnehin übliche Bestandteile üblicher Wasch- und Reinigungsmittel darstellen. Das pastenförmige Verfahrensprodukt wird im allgemeinen, was seinen pH-Wert betrifft, so belassen, wie es nach dem Bleichprozeß anfällt, d. h. die Paste besitzt einen pH-Wert zwischen 8 und 10. Für besondere Anwendungszwecke kann der pH-Wert durch Zugabe einer sauren Verbindung, deren Anwesenheit für die weitere Verwendung günstig zumindest aber unschädlich ist, auf Werte um den Neutralpunkt herabgesetzt werden. Geeignete saure Zusätze sind beispielsweise saure Salze, wie Natrium- oder Kaliumhydrogensulfat, anorganische Säuren wie Schwefelsäure oder organische Säuren wie Citronensäure oder Sulfonat- bzw. Sulfattenside in der Säureform.

Für eine längere Lagerzeit bzw. einen längeren Transport des pastenförmigen Reaktionsprodukts kann es von Bedeutung sein, daß mikrobielle Abbauprozesse wirksam verhindert werden. Zweckmäßigerweise enthält deshalb das erfindungsgemäß hergestellte pastenförmige Reaktionsprodukt einen die Lagerungsbeständigkeit verbessernden üblichen antimikrobiellen Zusatz in üblicher Menge. Dieser Zusatz besteht beispielsweise aus 0,1 bis 0,2 Gew.-% Glutardialdehyd.

Eine besonders bevorzugte Ausführungsform des Verfahrens zur Herstellung von hellfarbigen und farbstabilen Alkylglykosiden nach der Methode der Direktsynthese ist durch die Anwendung der folgenden kumulativen Verfahrensschritte gekennzeichnet:

1. Die Glykose, insbesondere die Glucose, wird im Alkohol mit hochtourigen Rührern oder mit anderen hochwirksamen technischen Mischvorrichtungen feindispergiert.

2. Die zur Neutralisation des Säurekatalysators, vorzugsweise einer Sulfonsäure, verwendete Base besteht ganz oder überwiegend aus Magnesiumoxid.

3. Die Basenmenge wird so berechnet, daß über die eigentliche Neutralisation hinaus eine basisch reagierende Mischung, vorzugsweise von pH 8 bis 10 erhalten wird.

4. Das Reaktionsgemisch wird nach der Neutralisation nicht filtriert.

5. Beim Abdestillieren des überschüssigen Alkohols im Vakuum wird schließlich auf eine Sumpftemperatur von 160 bis 180 °C erhitzt bzw. die Beheizungstemperatur im Verdampfergerät der zweiten Stufe auf etwa 170 °C bis 180 °C gebracht.

Die hohe Produktqualität des Endprodukts nach der Bleiche ist auf die kumulative Anwendung dieser Verfahrensschritte zusammen mit den anderen Verfahrensschritten zurückzuführen. Sinngemäß läßt sich diese Kombination von Verfahrensparametern auch bei den anderen Herstellungsverfahren für Alkylglykoside wie z. B. beim Umacetalisierungsverfahrens mit Butanol oder Propylenglykol, bzw. bei solchen Verfahren, die Polyglykosen, insbesondere Stärke und Stärkeabbauprodukte als Ausgangsstoffe benutzen, anwenden.

## Beispiele

Die folgenden Beispiele beschrieben das erfindungsgemäße Verfahren.

### Beispiel 1:

In diesem Beispiel wird das erfindungsgemäße Verfahren der Direktsynthese von $C_{12}$-Alkylglucosid im Labormaßstab nach der Methode der portionsweisen Zugabe einer Glucose/Fettalkoholsuspension (Slurry-Variante) beschrieben.

In einem 2-Liter-Dreihalskolben mit Rührer, Tropftrichter und Kolonne zur Wasserabscheidung wurden 559 g (3 Mol) n-Dodecanol und 2,2 g (11,2 mMol) Paratoluolsulfonsäure vorgelegt und auf 110 bis 114 °C erhitzt. Dann wurde eine Suspension von 180 g (1 Mol) wasserfreier Glucose (Puridex der Fa. Cerestar Deutschland GmbH) in weiteren 559 g (3 Mol) n-Dodecanol portionsweise, und zwar in 10 Portionen, in Abständen von 5 Minuten hinzudosiert. Dabei wurde vor der ersten Dosierung ein Vakuum, das zwischen 10 und 15 mbar schwankte, angelegt. Die Glucose/Fettalkoholsuspension war vor dem Dosieren ebenfalls auf ca. 110 °C erwärmt worden. Das Reaktionswasser wurde über den Destillationsaufsatz aus dem Reaktionsmilieu entfernt und in einer Kühlfalle, die mit flüssigem Stickstoff gekühlt war, ausgefroren und aufgefangen. Es wurden insgesamt 19 g Wasser gemessen. Im Anschluß daran wurde noch 120 Minuten ebenfalls bei 110 bis 115 °C nachgerührt. Das Reaktionsgemisch wurde dann auf 90 °C abgekühlt und bei Normaldruck mt 2,0 g (17,5 mMol) Magnesiumethylat versetzt. Die Mischung wurde 30 Minuten lang gerührt. Das Reaktionsgemisch hatte anschließend einen pH-Wert zwischen 9 und 10. Bei einem Vakuum von 0,1 bis 0,01 mbar und einer Sumpftemperatur von 120 bis 170 °C wurde der überschüssige Alkohol aus dem Reaktionskolben abdestilliert. Die Destillatmenge betrug 976 g; der Destillationsrückstand, d. h. das eigentliche Produkt fiel in einer Menge von 299,1 g an. Dieser Rückstand wurde bei einer Temperatur von 90 °C mit Wasser und 4,5g einer 35%igen $H_2O_2$-Lösung versetzt und so unter Rühren innerhalb von 120 Minuten zu einer 60%igen Paste verarbeitet. Während des Bleichprozesses wurde der pH-Wert kontrolliert und durch Hinzufügen von NaOH 50%ig auf dem Wert von pH 9 gehalten.

Produktkenndaten: Hydroxylzahl: 656; restlicher Fettalkohol: 0,7 Gew.-%; Dodecylmonoglucosid: 67 Gew.-%; Dodecyldiglucosid: 16 Gew.-%; Dodecyltriglucosid: 5 %; Dodecyltetraglucosid 2 Gew.-%; Dodecylpentaglucosid 1 Gew.-%; Polyglucose: 7 Gew.-%, Glucose unter 1 Gew.-%. Klettzahlen: nach der Bleiche: 20; nach dem Farbstabilitätstest: 25.

**Beispiel 2:**

Dieses Beispiel beschreibt die Herstellung eines $C_{12}/C_{14}$-Alkylglucosids aus wasserfreier Glucose und einem technischen Fettalkohol (Gemisch aus ca. 75 Gew.-% Dodecanol und ca. 25 Gew.-% Tetradecanol) nach der sogenannten Batch-Variante (Ansatz mit der Gesamtmenge der Reaktionskomponenten) im Kleintechnikumsmaßstab

In einem 150 Liter-Kessel aus Edelstahl wurden 25,0 kg (129 Mol) des Dodecanol/Tetradecanol-Gemisches (Lorol S, Henkel KGaA) und 7,7 kg (43 Mol) wasserfreie Glucose (Puridex) vorgelegt und auf etwa 80 °C unter Rühren aufgeheizt. Dann wurden 53 g (0,28 Mol) p-Toluolsulfonsäure hinzugegeben. Anschließend wurde das Reakti onsgemisch weiter auf ca. 115 °C aufgeheizt, wobei gleichzeitig ein Vakuum von ca. 20 mbar angelegt wurde. Unter diesen Bedingungen wurde während etwa 4 Stunden lang gerührt und dabei das Reaktionswasser im Vakuum abgezogen. Die resultierende gelblich trübe Reaktionslösung wurde auf 90 °C abgekühlt und bei Normaldruck mit 35 g (0,87 Mol) Magnesiumoxid versetzt. Daraufhin wurde 30 Minuten nachgerührt. In der Reaktionsmischung wurde ein pH-Wert von ca. 10, gemessen. Danach wurde bei einem Vakuum von 0,5 bis 1 mbar der Alkoholüberschuß abdestilliert, wobei man die Sumpftemperatur im Laufe von 3 Stunden auf 170 °C erhöhte. Während der insgesamt 3 Stunden lang dauernden Destillation, die im Reaktionskessel durchgeführt wurde, wurden ca. 20 kg Fettalkohol abdestilliert. Der Destillationsrückstand stellte eine orangerote klare Schmelze dar, die auf ca. 105 °C abgekühlt und dann mit entsalztem Wasser von 70 °C zu einer ca. 50%igen Paste vermischt wurde. Dann wurden 100 ml 50%ige Natronlauge als 1 Portion und 200 ml 35%iges Wasserstoffperoxid in 5 Portionen während 2 1/2 Stunden hinzugegeben. Danach wurde das Gemisch weitere 5 Stunden bei 80 °C gerührt. Als Reaktionsendprodukt wurden so 18,9 kg einer hellgelben transparenten Paste (49,1 % Wasser, pH-Wert 9 bis 10) erhalten.

Produktkenndaten: Hydroxylzahl: 694; restlicher Fettalkohol: 1,8 %; Monoglucosid: 51 Gew.-%; Diglucosid: 16 Gew.-%; Triglucosid: 6 Gew.-%; Tetraglucosid: 4 Gew.-%; Pentaglucosid: 2 Gew.-%; Hexaglucosid unter 1 Gew.-%; Polyglucose: ca. 17 Gew.-%; Salze unter 2 Gew.-%. Klettzahlen: 20/20. Nach 1/2jähriger Lagerung des Produkts waren die Farbzahlen und die Zusammensetzung (gaschromatographisch bestimmt) unverändert.

Zum Vergleich wurde die Farbstabilität eines nach den Lehren des Standes der Technik hergestellten Produkts bestimmt. Dieses Produkt wurde nach Beispiel 6 von US 3,839,318, Mansfield, mit einer Dodecanol/Tetradecanol-Mischung hergestellt, allerdings wurde zum Neutralisieren des saurem Katalysators (Schwefelsäure) nicht die wäßrige Natronlauge des Beispiels 6, sondern gemäß EP 132 046 B1 Natriummethylat als Base benutzt und der pH-Wert auf 7,0 eingestellt. Das so erhaltene Produkt hatte die Klettzahl 45 bzw. 125 nach dem Behandeln mit Alkali gemäß Farbstabilitätstest. Nach Überführen in eine 60%ige Paste und Bleichen mit $H_2O_2$ wurden die Klettzahlen 25 (direkt nach der Bleiche, pH-Wert kleiner als 7) bzw. 110 (nach Farbstabilitätstest) gemessen. Bei einer Wiederholung des Versuchs wurde der Destillationsrückstand lediglich auf 130 °C abgekühlt. Das resultierende Verfahrensprodukt hatte die gleichen Kenndaten.

## Beispiel 3:

Dieses Beispiel beschreibt die Herstellung von $C_{12}/C_{14}$-Alkylglucosid in einem großtechnischen Ansatz

Von der Gesamtmenge von 1860 kg $C_{12}/C_{14}$-Fettalkohol (Verteilung: Dodecanol ca. 75 Gew.%, Tetradecanol ca. 25 Gew.-%) wurde die Hälfte in einem 25 m³-Reaktor zusammen mit 300 kg wasserfreier Glucose (Puridex) zu einer Suspension verarbeitet. Die Feindispergierung der Suspension erfolgte mit Hilfe eines Stator/Rotor-Mischers, wobei sich die Suspension auf 75 °C erwärmte. In einem zweiten Reaktor (3,2 m³) mit Destillationskolonne und externem Flüssigkeitskreislauf, der aus einer Pumpe und einem Wärmeaustauscher bestand, wurde der restliche Fettalkohol zusammen mit 3,9 kg p-Toluolsulfonsäure vorgelegt und auf 115 °C erhitzt. Der Reaktor wurde dann auf einen Druck von 20 bis 30 mbar evakuiert. Dann wurde während 1 Stunde die Glucose/Fettalkohol-Suspension kontinuierlich hinzugegeben. Während dieser Zeit und einer Nachreaktionszeit von 2 Stunden wurden insgesamt 30 kg Wasser abdestilliert. Die zur Entfernung des Wassers und zur Aufrechterhaltung der Reaktionstemperatur notwendige Wärmeenergie wurde über den externen Flüssigkeitskreislauf in das Reaktionsgemisch eingebracht. Das Reaktionswasser wurde in einer gekühlten Vorlage aufgefangen und gemessen. Nach beendeter Reaktion wurde auf 90 °C abgekühlt. Dann wurden zur Neutralisation des sauren Katalysators 2,9 kg Magnesiumethylat in fester Form über den externen Flüssigkeitskreislauf eingesaugt. Danach wurde Normaldruck eingestellt.

Anschließend wurde das Reaktionsgemisch in einen Dünnschichtverdampfer Typ Sambay (0,75 qm Verdampferfläche, 8 mbar, ca. 170 °C) geleitet und der überschüssige Fettalkohol bis auf einen Abreicherungswert von ca. 32 % abgetrennt. Das bei 135 °C gehaltene Produkt war niedrig viskos und konnte leicht in einen Kurzwegverdampfer mit Rollenwischer vom Typ KD 75, Fa. Leybold, übergeführt werden. Der Kurzwegverdampfer wurde unter den folgenden Bedingungen betrieben: Verdampferfläche 0,75 qm; Arbeits-druck 0,075 mbar, im Verdampfer gemessen; Beheizungstemperatur 170 °C; Sumpfablauftemperatur 162 °C. Alternativ wurde in einem zweiten Ansatz ein Dünnschichtverdampfer auch in der zweiten Abreicherungsstufe verwendet. Das Produkt wurde chargenweise (90 kg) in geschmolzenem Zustand bei 150 °C in einem Druckkessel mit ca. 88 kg Wasser von Raumtemperatur versetzt, um so eine ca. So%ige Paste herzustellen. Separat wurden 1,3 kg einer

35%igen $H_2O_2$-Lösung und 0,9 kg einer 50%igen NaOH-Lösung zudosiert. Nach 3stündigem Rühren bei 90 °C wurde auf 50 °C abgekühlt.
Produktkenndaten: pH-Wert 9,5; Klettzahl 23 (nach der Bleiche) bzw. 26 nach dem Behandeln mit Alkali und in der Wärme nach dem Farbstilitätstest. Zusammensetzung des Produkts (wasserfrei): Hydroxylzahl 650; restlicher Fettalkohol 3 Gew.-%; Alkyimonoglucosid 62,8 Gew.-%; Alkyldiglucosid 15,4 Gew.-%; Alkyltriglucosid 5,8 Gew.-%; Alkyltetraglucosid 2,5 Gew.-%; Alkylpentaglucosid 1,1 Gew .-%; Alkylhexaglucosid 0,2 Gew.-%; Polyglucose 6 Gew.-%; Glucose weniger als 1 Gew. -%; Salze weniger als 2 Gew.-%.

## Ansprüche

1. Verfahren zur direkten Herstellung von Alkylglykosiden durch Acetalisierungsreaktion von höheren aliphatischen primären Alkoholen mit Glykosen, insbesondere mit Glucose, in Gegenwart eines sauren Katalysators, rascher Entfernung des Reaktionswassers, Neutralisation des Katalysators mit einer Base, Abdestillation des Alkoholüberschusses und Überführen des Reaktionsproduktes in eine wäßrige Paste und Bleichen dieser Paste, wobei der aliphatische Alkohol im molaren Überschuß zur Glykose eingesetzt wird und wobei die Bildung und Abführung des Reaktionswassers unter Vakuum erfolgt und Reaktionstemperaturen über 80 °C angewendet werden, dadurch gekennzeichnet, daß man:

a) Mischungen aus aliphatischem primärem Alkohol, Glykose und saurem Katalysator bei erhöhter Temperatur erzeugt und zur Reaktion bringt, wobei man entweder
(i) eine Teilmenge des Alkohols mit dem Katalysator vorlegt, die Mischung erwärmt, und dann eine erwärmte Suspension der Glykose in der restlichen Alkoholmenge portionsweise oder kontinuierlich zu der Alkohol/Katalysatormischung zudosiert und dabei im Vakuum das entstehende Reaktionswasser abdestilliert, oder
(ii) eine Mischung des gesamten Alkohols und der Glykose vorlegt, erwärmt und den sauren Katalysator zur erwärmten Mischung hinzugibt, dann ein Vakuum anlegt und weiter bis zum Einsetzen der Reaktion erwärmt und das entstehende Reaktionswasser abdestilliert,

b) dabei die Ansatzverhältnisse so wählt, daß das Molverhältnis Glykose zu aliphatischem Alkohol bei 1 : 2 bis 1 : 10, vorzugsweise 1 : 3 bis 1 : 6 liegt,

c) das Reaktionsgemisch bei dieser Temperatur und diesem Unterdruck so lange hält, vorzugsweise unter Durchmischung, bis das Reaktionswasser vollständig entfernt ist,

d) anschließend das Reaktionsgemisch auf ca. 90 ° abkühlt, dann eine organische oder anorganische basische Alkali-, Erdalkali- oder Aluminium- bzw. Alkali/Aluminiumverbindung in solchen Mengen hinzufügt, daß sich über die Neutralisation des sauren Katalysators hinaus ein pH-Wert von wenigstens 8, vorzugsweise 8 bis 10 einstellt, und vorzugsweise erst danach Normaldruck herstellt,

e) daß man vorzugsweise ohne vorherige Filtration den überschüssigen Alkohol aus dem alkalischen Gemisch im Vakuum auf an sich übliche, das Reaktionsprodukt schonende Weise auf einen Wert von unterhalb 5 Gew.-% des Reaktionsproduktes abdestilliert, und

f) daß man anschließend auf ca. 105 °C bis 130 °C abkühlt und durch Hinzugabe von Wasser eine 30- bis 70prozentige Paste erzeugt, die man durch vorzugsweise portionsweises Hinzufügen von Aktivsauerstoffverbindungen, vorzugsweise Wasserstoffperoxid, ca. 0,1 bis 5 Stunden bei ca. 80 °C rührt, wobei man gegebenenfalls durch Hinzufügen von Alkali, vorzugsweise Natronlauge, dafür sorgt, daß der pH-Wert während dieses Bleichprozesses bei 8 bis 10 bleibt.

2. Verfahren nach Anspruch 1, dadurch gekennzeichnet, daß man als höhere aliphatische primäre Alkohole solche mt 8 bis 20 Kohlenstoffatomen, vorzugsweise 12 bis 18 Kohlenstoffatomen einsetzt.

3. Verfahren nach den Ansprüchen 1 oder 2, dadurch gekennzeichnet, daß man als Teilmenge etwa 30 bis 70 Gew.-% des Alkohols zusammen mit dem Katalysator vorlegt, die Mischung auf etwa 100 bis 120 °C erwärmt, und dann die Glykose in erwärmter Suspension in der restlichen Alkoholmenge, vorzugsweise kontinuierlich unter Vakuum, hinzugibt und das entstehende Reaktionswasser abdestilliert.

4. Verfahren nach den Ansprüchen 1 oder 2, dadurch gekennzeichnet, daß man eine Mischung der gesamten Alkoholmenge und der Glykose vorlegt, erwärmt, dann den sauren Katalysator zu der erwärmten Mischung hinzugibt, ein leichtes Vakuum anlegt und weiter bis auf ca. 100 bis 120 °C erwärmt und das entstehende Reaktionswasser abdestilliert

5. Verfahren nach einem der Ansprüche 1 bis 4, dadurch gekennzeichnet, daß man die Glykose/Alkohol-Suspension einer Feindispergierung unterzieht.

6. Verfahren nach einem der Ansprüche 1 bis 5, dadurch gekennzeichnet, daß man das Vakuum so einstellt, daß der Siedepunkt des Alkohols um wenigstens 30 °C gesenkt wird, vorzugsweise daß man ein Vakuum von 10 bis 50 mbar einstellt.

7. Verfahren nach einem der Ansprüche 1 bis 6, dadurch gekennzeichnet, daß man als höhere aliphatische primäre Alkohole gesättigte $C_{12}$-$C_{18}$-Alkohole, die vorzugsweise geradkettig sind, einsetzt.

8. Verfahren nach einem der Ansprüche 1 bis 7, dadurch gekennzeichnet, daß man als saure Katalysatoren solche Verbindungen und in solchen Mengen verwendet, daß deren resultierende Alkali-, Erdalkali- bzw. Aluminium-Salze im Produkt verbleiben können, vorzugsweise eine Säure aus der Gruppe, bestehend aus Schwefelsäure, Phosphorsäure oder aliphatische und/oder aromatische Sulfonsäuren, vorzugsweise Paratoluolsulfonsäure, in einer Menge von vorzugsweise 0,005 bis 0,02 Mol pro Mol der eingesetzten Glykose.

9. Verfahren nach einem der Ansprüche 1 bis 8, dadurch gekennzeichnet, daß man als basische Substanzen für die Neutralisation des sauren Katalysators und darüber hinaus für die Einstellung eines basischen pH-Werts anorganische, feinpulvrisierte Verbindungen aus der Gruppe, bestehend aus Calciumhydroxid, Calciumoxid, Magnesiumhydroxid, Magnesiumoxid, die Zeolithe NaA oder NaX, vorzugsweise in Kombination mit Calciumhydroxid, und als organische Verbindungen die Alkoholate von niedrig siedenden Alkoholen, vorzugsweise $C_1$-$C_4$-Alkoholen, in Form der Alkalimetall- und/oder Erdalkalimetall-Verbindungen, einsetzt.

10. Verfahren nach einem der Ansprüche 1 bis 9, dadurch gekennzeichnet, daß man als anorganische basische Verbindung Magnesiumoxid und als organische basische Verbindung ein Magnesiumalkoholat, insbesondere Magnesiumethylat einsetzt.

11. Verfahren nach einem der Ansprüche 1 bis 10, dadurch gekennzeichnet, daß man nach der Neutralisation die basische Reaktionsmischung unter einem Vakuum, das zur Abdestillation des Alkoholüberschusses erforderlich ist, bis auf eine Sumpftemperatur von 160 bis 180 °C, insbesondere von 160 bis 170 °C erhitzt, wobei man auch bei Ansätzen mit kürzerkettigen Alkoholen mit entsprechend niederen Siedepunkten diese hohe Sumpftemperatur einstellt.

12. Verfahren nach Anspruch 1, gekennzeichnet durch die Anwendung der folgenden kumulativen Verfahrensschritte:

1. Die Glykose, insbesondere die Glucose, wird im Alkohol mit hochtourigen Rührern oder mit anderen hochwirksamen technischen Mischvorrichtungen feindispergiert.

2. Die zur Neutralisation des Säurekatalysators vorzugsweise einer Sulfonsäure, verwendete Base besteht ganz oder überwiegend aus Magnesiumoxid.

3. Die Basenmenge wird so berechnet, daß über die eigentliche Neutralisation hinaus eine basisch reagierende Mischung, vorzugsweise von pH 8 bis 10 erhalten wird.

4. Das Reaktionsgemisch wird nach der Neutralisation nicht filtriert.

5. Beim Abdestillieren des überschüssigen Alkohols im Vakuum wird schließlich auf eine Sumpftemperatur von 160 bis 180 °C erhitzt bzw. die Beheizungstemperatur im Verdampfergerät der zweiten Stufe auf etwa 170 °C bis 180 °C gebracht.

13. Alkylglykosid als Erzeugnis nach einem der Ansprüche 1 bis 12, dadurch gekennzeichnet, daß dessen Oligomerisierungsgrad bei 1 bis 5, vorzugsweise 1 bis 1,5, liegt und insbesondere so eingestellt wird, daß die Menge an Alkylmonoglykosid, bezogen auf die Gesamtmenge aus Alkylmonoglykosid und Alkyloligoglykosid, deutlich über 70 Gew.-% liegt.

14. Erzeugnis nach Anspruch 13, dadurch gekennzeichnet, daß die Restalkoholmenge, bezogen auf das wasserfreie Produkt, auf 0,2 bis 5, insbesondere 0,5 bis 2,5 Gew.-% eingestellt ist.

15. Erzeugnis nach den Ansprüchen 13 und 14, dadurch gekennzeichnet, daß es in Form einer wäßrigen Paste mit 30 bis 60 Gew.-% Wasseranteil vorliegt, welche die aus der Neutralisation des Katalysators und dem Bleichvorgang stammenden Salze enthält.

16. Erzeugnis in Form einer wäßrigen Paste nach einem der Ansprüche 13 bis 15 dadurch gekennzeichnet, daß es einen die Lagerungsbeständgket verbessernden antimikrobiellen Zusatz in Mengen von 0,1 bis 0,2 Gew.-% enthält.

**Europäisches Patentamt**

# EUROPÄISCHER RECHERCHENBERICHT

Nummer der Anmeldung

EP 89 11 7745

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | EP-A-0 132 046 (PROCTER & GAMBLE) <br> * Seite 9, Zeile 16 - Seite 11, Zeile 30; Anspruch 1 * <br> --- | 1 | C 07 H 15/04 |
| A,D | US-A-3 839 318 (R.C. MANSFIELD) <br> * Spalte 6, Zeilen 24-67; Anspruch 1 * <br> --- | 1 | |
| A,D | EP-A-0 165 721 (STALEY) <br> * Seite 16, Zeile 1 - Seite 18, Zeile 18; Ansprüche 1,2 * <br> ----- | 1 | |

**RECHERCHIERTE SACHGEBIETE (Int. Cl.5)**

C 07 H 15/00

Der vorliegende Recherchenbericht wurde für alle Patentansprüche erstellt

| Recherchenort | Abschlußdatum der Recherche | Prüfer |
|---|---|---|
| DEN HAAG | 27-11-1989 | BRENNAN J. |

EPO FORM 1503 03.82 (P0403)